(19) Europäisches Patentamt / European Patent Office / Office européen des brevets

(11) **EP 2 448 669 B1**

## (12) EUROPÄISCHE PATENTSCHRIFT

(45) Veröffentlichungstag und Bekanntmachung des Hinweises auf die Patenterteilung:
**11.04.2018 Patentblatt 2018/15**

(21) Anmeldenummer: **10724792.6**

(22) Anmeldetag: **07.06.2010**

(51) Int Cl.:
**B01J 23/38** *(2006.01)*    **C07C 45/38** *(2006.01)*

(86) Internationale Anmeldenummer:
**PCT/EP2010/057911**

(87) Internationale Veröffentlichungsnummer:
**WO 2011/000668 (06.01.2011 Gazette 2011/01)**

(54) **GETRÄGERTER EDELMETALLHALTIGER KATALYSATOR ZUR OXIDATIVEN DEHYDRIERUNG ODER EPOXIDATION**

SUPPORTED CATALYST COMPRISING NOBLE METALS FOR OXIDATIVE DEHYDROGENATION OR EPOXIDATION

CATALYSEUR SUPPORTÉ CONTENANT UN MÉTAL NOBLE DESTINÉ À LA DÉSHYDROGÉNATION OXYDATIVE OU À L ÉPOXYDATION

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **02.07.2009 EP 09164422**

(43) Veröffentlichungstag der Anmeldung:
**09.05.2012 Patentblatt 2012/19**

(73) Patentinhaber: **BASF SE**
**67056 Ludwigshafen am Rhein (DE)**

(72) Erfinder:
• SEEBER, Georg
  67245 Lambsheim (DE)
• LÖCHER, Peter
  67550 Worms-Rheindürkheim (DE)
• BAUER, Stefan
  74889 Sinsheim (DE)
• ROSENDAHL, Tobias
  68259 Mannheim (DE)
• MÄURER, Torsten
  67245 Lambsheim (DE)
• WEGNER, Günter
  67354 Römerberg (DE)
• KAMASZ, Martin
  68307 Mannheim (DE)

(56) Entgegenhaltungen:
EP-A1- 0 112 261     EP-A1- 0 881 206
EP-A1- 1 209 121     DE-A1- 4 010 182
GB-A- 940 710     US-A- 4 816 606

• ABAD A ET AL: "Catalyst Parameters Determining Activity and Selectivity of Supported Gold Nanoparticles for the Aerobic Oxidation of Alcohols: The Molecular Reaction Mechanism" CHEMISTRY - A EUROPEAN JOURNAL, WILEY - V C H VERLAG GMBH & CO. KGAA, WEINHEIM, DE LNKD- DOI:10.1002/CHEM.200701263, Bd. 14, Nr. 1, 23. November 2007 (2007-11-23), Seiten 212-222, XP002536256 ISSN: 0947-6539

Bemerkungen:
Die Akte enthält technische Angaben, die nach dem Eingang der Anmeldung eingereicht wurden und die nicht in dieser Patentschrift enthalten sind.

**Beschreibung**

Die vorliegende Erfindung betrifft einen geträgerten Edelmetallkatalysator, ein Verfahren zu dessen Herstellung, sowie dessen Verwendung zur Epoxidierung oder oxidativen Dehydrierung, insbesondere zur Herstellung von olefinisch ungesättigten Carbonylverbindungen aus olefinisch ungesättigten Alkoholen durch oxidative Dehydrierung.

Ferner betrifft die vorliegende Erfindung die neue Verwendung kolloidalen Silbers zur Herstellung geträgerter silberhaltiger Katalysatoren, insbesondere für die Epoxidation oder oxidative Dehydrierung.

Insbesondere betrifft die vorliegende Erfindung die Verwendung von nach einem bestimmten Verfahren erhältlichen geträgerten Edelmetallkatalysatoren zur Herstellung von 3-Methylbut-2-en-1-al (MBA) aus 3-Methylbut-3-en-1-ol (MBE).

Die Herstellung von alpha-beta-ungesättigten Carbonylverbindungen durch oxidative Dehydrierung an geeigneten Katalysatoren ist dem Fachmann bekannt und vielfach in der Literatur beschrieben.

In der DE-B-20 20 865 wird dementsprechend ein Verfahren zur Herstellung von alpha-beta-ungesättigten Carbonylverbindungen beschrieben, wobei als Dehydrierungskatalysatoren ausweislich der Beschreibung Legierungen und Metallverbindungen, speziell einige Metalloxide der Nebengruppenelemente eingesetzt werden können. Weiterhin wird in dieser Schrift ausgeführt, dass die Katalysatoren in reiner Form wie in Form von Mischkatalysatoren mit oder ohne Trägersubstanz verwendet werden können. Als besonders geeignet werden Zinkoxid, Kadmiumoxid und Manganoxid sowie Mischkatalysatoren aus den Metallen Cu, Ag und/oder Zn genannt. Zur Herstellung des Katalysators finden sich in dieser Schrift keine weiteren Angaben.

In der EP-A 881 206 wird ein Verfahren zur kontinuierlichen technischen Herstellung ungesättigter aliphatischer Aldehyde in einem Rohrbündelreaktor beschrieben. Als bevorzugte Katalysatoren für dieses Verfahren werden Silber-Trägerkatalysatoren genannt, die aus Kugeln eines inerten Trägermaterials bestehen, die mit 0,1 bis 20 Gew.-%, bezogen auf die Menge des Trägers, einer Schicht aus metallischem Silber in Form einer glatten, abriebfesten Schale beschichtet sind. Weiterhin soll ein bestimmtes Verhältnis des größten Durchmessers der beschichteten Katalysatorkugeln zum Innendurchmesser des Reaktionsrohrs vorzugsweise eingehalten werden.

Aus der DE-A 27 15 209 ist ein Verfahren zur Herstellung von 3-Atkylbuten-1-alen bekannt, wobei ein Katalysator mit einer Gesamtschichtdicke von 5 bis 35 mm und 2 oder mehr Schichten Silber- und/oder Kupferkristallen eingesetzt wird. Die Herstellung des Katalysators mit mehreren Schichten des Edelmetalls ist dabei relativ aufwändig.

Aus der EP-A 357 292 ist ein Verfahren zur Herstellung von Ethylenoxid bekannt. Als Katalysatoren in diesem Verfahren werden Silberkatalysatoren eingesetzt, wobei das Silber auf einem porösen hitzebeständigen Träger mit einer bestimmten spezifischen Oberfläche nach BET aufgebracht ist. Ausweislich den Angaben in dieser Schrift kann das Silber auf den Träger als Suspension von Silber oder Silberoxid in einem flüssigen Medium, beispielsweise Wasser, oder durch Tränkung des Trägers mit einer Lösung einer Silberverbindung aufgebracht werden. Anschließend wird diese Silberverbindung zu elementarem Silber durch thermische Behandlung reduziert. Auf eine mögliche Verwendung der so hergestellten silberhaltigen geträgerten Katalysatoren zur Herstellung von ethylenisch ungesättigten Carbonylverbindungen finden sich in dieser Schrift keine Hinweise, ebensowenig auf die Anwendungsmöglichkeiten von kolloidalem Silber.

Aus der EP-A 619 142 sind Silberkatalysatoren zur Oxidation von Ethylen zu Ethylenoxid bekannt, die durch Imprägnierung mit wässriger (kolloidaler) Lösung eines Silbersalzes erhalten werden; Hinweise auf Anwendungsmöglichkeiten von kolloidalem Silber gehen nicht aus diesem Dokument hervor.

Ferner sind aus der nicht vorveröffentlichten Deutschen Patentanmeldung DE 10 2008 014 910.1 edelmetallhaltige Katalysatoren bekannt, die durch Aufbringen einer komplexierten schwerlöslichen Verbindung eines Edelmetalls aus Suspension bzw. Lösung auf einen Träger und anschließende thermische Behandlung erhalten werden, bekannt.

Ähnliche Katalysatoren bzw. Verfahren sind z.B. auch bekannt aus GB 940 710 A, DE 40 10 182 A1, EP 1 209 121 A1, EP 0 112 261 A1, US 4,816,606 oder Abad et al, "Catalyst Parameters Determining Activity and Selectivity of Supported Gold Nanoparticles for the Aerobic Oxidation of Alcohols: The Molecular Reaction Mechnism", Chem. Eur. J. 2008, 14, 212-222.

3-Methylbut-2-en-1-al, auch unter dem Trivialnamen Prenal bekannt, ist eine wichtige Vorstufe für Citral, welches wiederum ein wichtiges Produkt für eine Vielzahl von chemischen Synthesen darstellt. Die in der Literatur beschriebenen Katalysatoren zur Herstellung von Prenal (MBA, 3-Methylbut-2-en-1-al) werden nach relativ komplexen Verfahren und unter Produktionsbedingungen hergestellt, die insgesamt verbesserungsbedürftig sind. Wünschenswert wäre es daher, edelmetallhaltige Trägerkatalysatoren für die Synthese von Prenal aus Isoprenol (MBE, 3-Methylbut-3-en-1-ol) zu erhalten, die auf einfache Weise zugänglich sind und auch hinsichtlich ihrer Selektivität einfach durch Zusätze von als Promotoren wirkenden Verbindungen gesteuert werden können.

Weiterhin wäre es wünschenswert auch für die Herstellung von Ethylenoxid aus Ethylen Katalysatoren zur Verfügung zu haben, die einfach herzustellen sind und dabei sehr gute Ausbeuten, Umsetzungen, Selektivitäten etc. aufweisen.

Aufgabe der vorliegenden Erfindung war es demgemäß, den Nachteilen und Erfordernissen, die sich aus dem bisherigen Stand der Technik ergeben, zu begegnen und entsprechende Katalysatoren bzw. Herstellungsverfahren für diese und Verwendungen zugänglich zu machen.

Erfindungsgemäß wird diese Aufgabe durch mit Steatit geträgerte silberhaltige Katalysatoren, die durch Aufbringen von kolloidalem Silber in Form einer kolloidalen Lösung, ggf. in Mischung mit als Promotoren wirkenden Zusätzen, auf Steatit als Trägermaterial und anschließende thermische Behandlung des erhaltenen Produkts hergestellt werden, gelöst.

Die vorliegende Erfindung betrifft in einem weiteren die Aufgabe lösenden Aspekt weiterhin ein Verfahren zur Herstellung dieses geträgerten edelmetallhaltigen Katalysators zur Epoxidation oder oxidativen Dehydrierung, wobei kolloidales Silber in Form einer kolloidalen Lösung, ggf. in Mischung mit als Promotoren wirkenden Zusätzen, auf Steatit als Trägermaterial aufgebracht wird und anschließend das erhaltene Produkt einer thermischen Behandlung unterworfen wird.

Ebenso betrifft die vorliegende Erfindung in einem weiteren die Aufgabe lösenden Aspekt die Verwendung der geträgerten edelmetallhaltigen Katalysatoren, die durch Aufbringen von kolloidalem Silber in Form einer kolloidalen Lösung, ggf. in Mischung mit als Promotoren wirkenden Zusätzen, auf Steatit als Trägermaterial und anschließende thermische Behandlung des erhaltenen Produkts hergestellt werden, zur Epoxidation oder oxidativen Dehydrierung, insbesondere zur Herstellung von olefinisch ungesättigten Carbonylverbindungen aus olefinisch ungesättigten Alkoholen durch oxidative Dehydrierung.

Im Rahmen der vorliegenden Erfindung sind alle Mengenangaben, sofern nicht anders angegeben, als Gewichtsangaben zu verstehen.

Im Rahmen der vorliegenden Erfindung bedeutet der Begriff "Raumtemperatur" eine Temperatur von 23°C. Temperaturangaben sind, soweit nicht anders angegeben, in Grad Celsius (°C).

Sofern nichts anderes angegeben wird, werden die angeführten Reaktionen bzw. Verfahrensschritte bei Normaldruck (Atmosphärendruck) durchgeführt.

Kugelförmig bedeutet im Rahmen der vorliegenden Erfindung, dass die betreffenden Primärpartikel sphäroid sind und in der Transmissionselektronenmikroskopie (TEM) vergleichbar mit idealen Kugeln keine Vorzugsrichtung oder-kanten zeigen.

Der Begriff "thermische Behandlung" steht im Rahmen der vorliegenden Erfindung für

a) Trocknung oder
b) Trocknung und Kalzinierung,

bzw. direktes Erhitzen auf Kalzinierungstemperatur und Kalzinieren.

Unter Kolloid sollen im Rahmen der vorliegenden Erfindung Teilchen bezeichnet werden, die in einem Lösungsmittel fein verteilt sind. Die mittlere Teilchengröße eines Kolloids liegt im Bereich zwischen etwa 1 nm (Nanometer) und 1000 nm (1 Mikrometer). Bei kugelförmigen Kolloiden wird unter mittlerer Teilchengröße der Gewichtsmittelwert der Teilchengröße verstanden; bei Kolloiden, die keine Kugelform aufweisen, kann sich die Angabe der Teilchengröße auch auf nur eine Dimension beziehen (z.B. bei plättchenförmigen Kolloiden).

Kolloidale Lösungen stehen in ihren Eigenschaften zwischen echten (moleculardispersen) Lösungen und Suspensionen (grobdisperse Lösungen) und kommen in ihren Eigenschaften den echten Lösungen umso näher, je kleiner die mittlere Teilchengröße des Kolloids ist.

Kolloidale Lösungen unterscheiden sich demgemäß in ihren Eigenschaften von Suspensionen.

Das Gewichtsmittel der Teilchengrößen, kann mittels einer analytischen Ultrazentrifuge entsprechend der Methode von W. Scholtan und H. Lange, Kolloid-Z. und Z.-Polymere 250 (1972), Seiten 782 bis 796, bestimmt werden. Die Ultrazentrifugenmessung liefert die integrale Massenverteilung des Teilchendurchmessers einer Probe. Hieraus lässt sich entnehmen, wie viel Gewichtsprozent der Teilchen einen Durchmesser gleich oder kleiner einer bestimmten Größe haben. Der mittlere Teilchendurchmesser, der auch als D50-Wert der integralen Massenverteilung bezeichnet wird, ist dabei als der Teilchendurchmesser definiert, bei dem 50 Gew.-% der Teilchen einen kleineren Durchmesser haben als der Durchmesser, der dem D50-Wert entspricht. Ebenso haben dann 50 Gew.-% der Teilchen einen größeren Durchmesser als der D50-Wert.

Kolloidale Systeme zeigen aufgrund ihrer im Verhältnis zum Volumen überdurchschnittlich großen Oberfläche ausgeprägte Effekte der Oberflächen-und Grenzflächenchemie.

Die Wechselwirkungen zwischen den einzelnen Kolloidteilchen in einem kolloiden System lassen sich durch Auswahl der Teilchen, Behandlung der Oberfläche und der Zusammensetzung der Flüssigkeit in weiten Bereichen beeinflussen.

Im Rahmen der vorliegenden Erfindungen wird kolloidal gelöstes Silber, d.h. kolloidale Lösungen von Silber, bei der Herstellung geträgerter Katalysatoren verwendet.

Die erfindungsgemäßen geträgerten edelmetallhaltigen Katalysatoren weisen einen spezifischen Widerstand gemessen bei 23°C von nicht mehr als 1000 Ohmmeter ($\Omega$*m), vorzugsweise von nicht mehr als 500 Ohmmeter ($\Omega$*m)

und besonders bevorzugt von nicht mehr als 100 Ohmmeter ($\Omega$*m) auf.

Der spezifische Widerstand wird im Rahmen der vorliegenden Erfindung in einer Messzelle ermittelt, deren Boden aus Edelstahl und deren Mantel aus isolierendem Kunststoff besteht (Innendurchmesser 10 mm, Höhe 32 cm, Katalysatorvolumen etwa 25 ml). Der Katalysator wird eingefüllt und etwas gerüttelt, um eine gleichmäßige Katalysatorschüttung zu erreichen. Danach wird ein Edelstahlstempel auf die Katalysatorschüttung aufgesetzt. Stempel und Boden dienen in dieser Messanordnung als Messelektroden. Zur Widerstandsmessung wird ein Strommessgerät mit der Probe in Reihe geschaltet und mit einem Netzgerät eine Spannung zwischen 10 mV und 5V eingestellt. Der zugehörige Strom wird registriert und der spezifische Widerstand wird berechnet. Die Messung wird bei atmosphärischem Druck und einer Luftfeuchtigkeit von max. 50% bei einer Temperatur von 23°C durchgeführt.

Bevorzugte Ausführungsformen der Erfindung sind den Unteransprüchen und der nachfolgenden Beschreibung und den Beispielen zu entnehmen.

Bei der erfindungsgemäßen Verwendung wird ein geträgerter edelmetallhaltiger Katalysator eingesetzt, welcher dadurch erhältlich ist, dass kolloidales Silber in Form einer kolloidalen Lösung, ggf. in Mischung mit als Promotoren wirkenden Zusätzen, auf Steatit als Trägermaterial aufgebracht und anschließend das erhaltene Produkt einer thermischen Behandlung unterworfen wird.

Kolloidales Gold bzw. kolloidales Silber sind seit langem bekannt und in der Literatur beschrieben.

Kolloidales Silber wurde in der Vergangenheit auch als Therapeutikum zur Infektionsbekämpfung eingesetzt, bevor wirksamere Mittel zur Verfügung standen. Derartige Produkte werden in der Literatur teilweise auch als Silbersol oder Silberwasser bezeichnet.

In der Regel handelt es sich bei kolloidalem Silber um flüssige Systeme elementaren Silbers oder schwerlöslicher Silberverbindungen, bevorzugt elementaren Silbers, im Lösungsmittel mit Teilchengrößen im Bereich von 1 bis 100, vorzugsweise von 10 bis 80 nm (Nanometer), wobei sich die Teilchengrößen auf elementares Silber bzw. Silberverbindungen beziehen. In den einzelnen Kolloidteilchen sind etwa 1000 bis 1000000000 Silberatome oder Moleküle der entsprechenden Silberverbindung enthalten.

Die Herstellung von kolloidalem Silber kann beispielsweise durch mechanisches Zermahlen in sogenannten Kolloidmühlen, durch verschiedene elektrolytische Verfahren (beispielsweise Abscheidung aus Elektroden) oder durch chemische Verfahren (beispielsweise die Reduktion bestimmter Silberverbindungen hergestellt werden.

Geeignete kolloidale Edelmetalle der vorstehend beschriebenen Art sind kommerziell erhältlich (z.B. Sigma-Aldrich, VWR, Fluka), wobei das Angebot für kolloidales Silber bzw. kolloidales Gold am umfangreichsten ist.

Die kolloidalen Edelmetallteilchen werden in das Lösungsmittel eingebracht, indem sie zum Lösungsmittel gegeben und dann verrührt werden. Dies kann bevorzugt bei Raumtemperatur und Normaldruck erfolgen; es ist jedoch ebenso möglich Temperatur und Druck den jeweiligen Bedürfnissen anzupassen.

In einer Variante der vorliegende Erfindung sind organische Lösungsmittel verwendbar, insbesondere $C_1$-$C_e$-Alkanole, Dimethylsulfid, Dimethylsulfoxid, N-Methylformamid, Dimethylformamid, Tetrahydrofuran, Aceton, Benzen, Toluen.

In einer weiteren Variante können wassermischbare Lösungsmittel, insbesondere gut wassermischbare wie Aceton, $C_1$-$C_6$-Alkanole, Dimethylsulfoxid, Dimethylformamid, Tetrahydrofuran, N-Methylformamid, in Mischung mit Wasser als Lösungsmittel eingesetzt werden.

Höchst bevorzugt wird im Rahmen der vorliegenden Erfindung als Lösungsmittel für das kolloidale Silber Wasser verwendet.

Das Silber liegt in den Lösungen, aus denen es auf das Trägermaterial aufgebracht wird, vorzugsweise in Anteilen, berechnet als Edelmetall bezogen auf die gesamte Lösung, im Bereich von 5 bis 35 Gew.-%, vorzugsweise im Bereich von 10 bis 30 Gew.-% und besonders bevorzugt im Bereich von 15 bis 25 Gew.-% vor.

Dieser Lösung des kolloidalen Silbers können als Promotoren geeignete weitere Zusätze zugesetzt werden. Nur beispielhaft seien hier Alkali-, Erdalkali- und Übergangsmetalle (wie Li, Rb, Cs, Ca, Mg, V, Co, Ni, Ir oder Re) erwähnt, die beispielsweise als Halogenide (Fluoride, Chloride) Carboxylate oder Nitrate oder auch in Form schwefelhaltiger Anionen wie Sulfate, Sulfite oder Sulfide eingesetzt werden können. Ebenfalls geeignet sind Phosphate, Cyanide und Hydroxide sowie Carbonate oder deren Mischungen. Schließlich können auch Anionen von Heteropolysäuren, insbesondere von Heteropolysäuren der Elemente der sechsten und siebten Nebengruppe des Periodensystems (Notation gemäß IUPAC-Vorschlag von 1985) eingesetzt werden. Die vorstehend genannten Promotoren können auch getrennt von den kolloiden Lösungen des Silbers eingesetzt werden. Art und Verfahren des Einsatzes solcher Promotoren in geträgerten Katalysatorsystemen sind dem Fachmann an sich bekannt und in der Literatur beschrieben, so dass sich hier nähere Angaben erübrigen.

Geeignete Steatit-Trägermaterialien sind dem Fachmann an sich bekannt und zum Teil kommerziell erhältlich von z.B. CeramTec und darüber hinaus in der Literatur beschrieben, auf die hier wegen näherer Einzelheiten verwiesen wird.

Trägermaterialien der vorliegenden Erfindung sind Steatite.

Insbesondere geeignet sind solche Träger, die in Kugelform vorliegen, wobei die kugelförmigen Trägerteilchen einen durchschnittlichen Durchmesser zwischen 0,5 und 3 mm aufweisen.

Dabei ist es erfindungsgemäß bevorzugt, für die Reaktion von MBE zu MBA Trägermaterial in Kugelform zu verwenden, wobei die kugelförmigen Trägerteilchen einen durchschnittlichen Durchmesser zwischen 0,5 und 1,5 mm aufweisen, wohingegen für die Epoxidierung, insbesondere von Ethylen, die Trägermaterialien bevorzugt die Form von Hohlringen haben. Bevorzugt sind Geometrien von 5-10 x 5-10 x 2-5 mm, insbesondere 6 x 6 x 3 oder 8 x 8 x 3 mm (Außendurchmesser mal Länge mal Lochdurchmesser des Hohlrings.

Gleichwohl ist die genaue Größe der Träger für die vorliegende Erfindung nicht kritisch.

In einer bevorzugten Ausgestaltung der vorliegenden Erfindung sind die Trägermaterialien für die Reaktion von MBE zu MBA möglichst wenig porös und weisen eine BET-Oberfläche von maximal 1 $m^2$/g, bevorzugt maximal 0,5 $m^2$/g auf und bestehen dabei aus Steatit.

In einer bevorzugten Ausgestaltung der vorliegenden Erfindung sind die Trägermaterialien für die Epoxidierung, insbesondere von Ethylen, möglichst wenig porös und weisen eine BET-Oberfläche von kleiner 10, bevorzugt kleiner 3 und insbesondere bevorzugt kleiner 1 $m^2$/g auf.

Die BET-Oberfläche der erfindungsgemäß einsetzbaren Träger kann dabei so gering sein, dass sie hinunter bis zu 0,01 $mm^2$/g, oder hinunter bis zu 0,001 $m^2$/g beträgt.

Steatit ist ein keramischer Werkstoff auf der Basis natürlicher Rohstoffe, der aus der Hauptkomponente Speckstein ($Mg(Si_4O_{10})(OH)_2$), einem natürlichem Magnesiumsilikat, besteht. Weiterhin können Zusätze von Ton und Feldspat oder Bariumcarbonat enthalten sein.

Die Herstellung der edelmetallhaltigen Katalysatoren gemäß der vorliegenden Erfindung umfaßt dabei die folgenden Schritte:

a) Aufbringen von kolloidalem Silber in Form einer kolloidalen Lösung, ggf. in Mischung mit als Promotoren wirkenden Zusätzen, auf Steatit als Trägermaterial,
b1) Trocknen des erhaltenen Produkts bei 150 bis 350°C, oder
b2) Trocknen des erhaltenen Produkts bei 150 bis 350°C und anschließende Kalzinierung bei 350 bis 550°C, wobei

Schritt a) die Teilschritte

a1) Vermischen des Trägermaterials mit kolloidal gelöstem Silber,
a2) gegebenenfalls Alterung der resultierenden Mischung durch Stehenlassen für einen Zeitraum von 1 bis 90, bevorzugt 30 bis 60, besonders bevorzugt 40 bis 50 Minuten, gegebenenfalls unter kontinuierlichem oder zeitweisem Rühren
a3) Abtrennen des erhaltenen Produkts, bevorzugt durch Filtrieren und/oder Abdampfen,

umfassen kann.

Die in Schritt a2) gegebenenfalls durchgeführte Alterung wird optional durchgeführt, falls die daraus resultierenden Eigenschaftsänderungen gewünscht sind.

Das in Schritt a3) gegebenenfalls durchgeführte Abdampfen stellt eine Vortrocknung dar und findet im allgemeinen bei Temperaturen zwischen 15 und 80°C, sowie unter normalem Atmosphärendruck statt. Das Abdampfen kann zusammen mit dem Filtrieren erfolgen.

Es ist im Rahmen der vorliegenden Erfindung möglich, Schritte a3) und b1) oder b2) kontinuierlich ineinander übergehen zu lassen, so dass keine Wartezeit zwischen diesen Schritten eingehalten wird.

Es ist aber ebenso möglich eine Wartezeit zwischen diesen Schritten einzuhalten. Die Dauer der Wartezeit ist aber unkritisch und bestimmt sich lediglich aus den jeweiligen praktischen Umständen. In einer bevorzugten Ausgestaltung liegt diese zwischen 1 und 120 Minuten,

Die in Schritten b1) und b2) angeführten Alternativen bzw. das direkte Erhitzen auf Kalzinierungstemperatur und anschließende Kalzinierung werden im Rahmen der vorliegenden Erfindung zusammenfassend auch als "thermische Behandlung" bezeichnet.

In Schritt b2) kann zwischen den einzelnen Schritten "Trocknung" und "Kalzinierung" eine Wartezeit eingehalten werden. Die Dauer der Wartezeit ist aber unkritisch und bestimmt sich lediglich aus den jeweiligen praktischen Umständen. Bevorzugt liegt diese zwischen1 und 300 Minuten.

In einer Variante der vorliegenden Erfindung ist es möglich, nach der Trocknung das Produkt zu lagern und erst nach einem beliebigen Zeitraum (auch nach Monaten) zu kalzinieren, die Katalysatorperformance wird dadurch nicht beeinträchtigt.

Es ist im Rahmen der vorliegenden Erfindung in einer Variante möglich, die in Schritt b2) erwähnten Schritte "Trocknung" und "Kalzinierung" kontinuierlich ineinander übergehen zu lassen.

Das in Schritt a) erhaltene Produkt kann in einer Variante der vorliegenden Erfindung direkt auf die Kalzinierungstemperatur erhitzt werden, so dass die Trocknung quasi "im Schnelldurchlauf" erfolgt.

Die Herstellung der edelmetallhaltigen Katalysatoren gemäß der vorliegenden Erfindung umfaßt dabei in dieser Variante die folgenden Schritte

a) Aufbringen von kolloidalem Silber in Form einer kolloidalen Lösung, gegebenenfalls in Mischung mit als Promotoren wirkenden Zusätzen, auf Steatit als Trägermaterial und

anschließende Kalzinierung bei 350 bis 550°C,
wobei das in a) erhaltene Produkt direkt auf Kalzinierungstemperatur erhitzt wird.

Die Variante des Schrittes b1) wird in einer bevorzugten Ausgestaltung angewendet, wenn der Katalysator zur Epoxidierung, insbesondere zur Herstellung von Ethylenoxid aus Ethylen, verwendet werden soll.

Die Variante des Schrittes b2) wird in einer anderen bevorzugten Ausgestaltung angewendet, wenn der Katalysator zur oxidativen Dehydrierung, insbesondere der Herstellung von MBA aus MBE, verwendet werden soll.

In einer Variante der vorliegende Erfindung umfaßt die Herstellung keine weiteren Schritte, sondern besteht aus den genannten.

Dieses Herstellungsverfahren betrifft sowohl die erfindungsgemäßen edelmetallhaltigen Katalysatoren, als auch die erfindungsgemäße Verwendung zur Herstellung der edelmetallhaltigen Katalysatoren und selbstverständlich das erfindungsgemäße Verfahren.

Nach dem Aufbringen des kolloidalen Silbers, gegebenenfalls mit dem Zusatz von Promotoren, aus Lösung auf das Trägermaterial wird dieses in einer ersten erfindungsgemäßen Variante einer Trocknung bei Temperaturen im Bereich von 150 bis 350°C, vorzugsweise von 200 bis 300°C und besonders bevorzugt von 225 bis 285°C unterworfen.

In einer zweiten erfindungsgemäßen Variante wird nach dem Aufbringen des kolloidalen Edelmetalls, gegebenenfalls mit dem Zusatz von Promotoren, aus Lösung auf das Trägermaterial, dieses einer Trocknung bei Temperaturen im Bereich von 150 bis 350°C, vorzugsweise von 200 bis 300°C und besonders bevorzugt von 225 bis 285°C und anschließend einer Kalzinierung bei Temperaturen im Bereich von 350 bis 550°C, vorzugsweise von 400 bis 500°C und besonders bevorzugt von 425 bis 475°C unterworfen.

Die Trocknung erfolgt in beiden Varianten bevorzugt für einen Zeitraum im Bereich von 2 bis 45 Minuten, vorzugsweise 5 bis 30 Minuten und besonders bevorzugt 10 bis 20 Minuten.

Die Kalzinierung erfolgt bevorzugt für einen Zeitraum im Bereich von 15 Minuten bis 3 Stunden, vorzugsweise 30 Minuten bis 2 Stunden und besonders bevorzugt 45 bis 90 Minuten.

Die Trocknung erfolgt dabei bevorzugt unter üblichen Nebenbedingungen, d.h. bei Atmosphärendruck (oder einem geringen apparativ bedingten Überdruck - durch den Gaseintrag in die Apparatur entsteht ein geringer Rückstau, der Druck erzeugt) unter Stickstoff-, Edelgas- oder Luftatmosphäre, bevorzugt Luftatmosphäre.

Die Kalzinierung erfolgt dabei bevorzugt unter üblichen Nebenbedingungen, d.h. bei Atmosphärendruck (oder einem geringen apparativ bedingten Überdruck - durch den Gaseintrag in die Apparatur entsteht ein geringer Rückstau, der Druck erzeugt) unter Stickstoff-, Edelgas- oder Luftatmosphäre, bevorzugt Luftatmosphäre.

Durch die thermische Behandlung wird auf der Oberfläche des Trägermaterials, wobei die Oberfläche auch die innere Oberfläche der Poren umfaßt, aus dem kolloidalen Silber eine Beschichtung von Silber selbst gebildet, welche dann die aktive Spezies des geträgerten Katalysators darstellt.

Die Silbergehalte, gemessen in Gew.-%, bezogen auf den Katalysator (also Träger plus Edelmetall), liegen nach der thermischen Behandlung im allgemeinen im Bereich von 0,5 bis 40 Gew.-%, vorzugsweise im Bereich von 0,8 bis 30 Gew.-% und besonders bevorzugt im Bereich von 1,7 bis 20 Gew.-%.

Dabei hat es sich in manchen Fällen als vorteilhaft herausgestellt, wenn unterschiedliche Träger unterschiedlichen Gehalte an Edelmetall aufweisen.

Für den Fall, dass der Katalysator zur oxidativen Dehydrierung, insbesondere zur Herstellung von MBA aus MBE, eingesetzt werden soll, ist es erfindungsgemäß bevorzugt, wenn die Silbergehalte im Bereich von 0,5 bis 6,0, insbesondere 0,8 bis 3, Gew.-%, bezogen auf den Katalysator, liegen.

Für den Fall, dass der Katalysator zur Epoxidierung, insbesondere zur Herstellung von Ethylenoxid aus Ethylen, eingesetzt werden soll, ist es erfindungsgemäß bevorzugt, wenn die Silbergehalte im Bereich von 5 bis 40, insbesondere 10 bis 30, Gew.-%, bezogen auf den Katalysator, liegen.

Insbesondere wenn Steatit mit einer BET-Oberfläche von kleiner 1 $m^2$/g als Trägermaterial eingesetzt wird, liegt der Silbergehalt, bezogen auf den Katalysator, bevorzugt im Bereich von 0,5 bis 2,5 Gew.-%, besonders bevorzugt im Bereich von 0,8 bis 2,2 Gew.-% und insbesondere im Bereich von 1,7 bis 2,2 Gew.-%.

Den genauen Gehalt an Silber kann der Fachmann aufgrund seines Fachwissens durch einfache Maßnahmen, wie Edelmetallgehalt der kolloidalen Lösung, Verweildauer des Trägers in der kolloidalen Lösung und Größe bzw. Beschaffenheit des Trägers variieren und an die jeweilige Aufgabe anpassen. Beispielsweise kann bei porösen Trägern dadurch, dass die Wasseraufnahme in die Poren des Trägermaterials eine Rolle spielt, die Imprägnierlösung exakt auf den Träger zugeschnitten werden, wohingegen bei nichtporösen Trägern der Edelmetallgehalt hauptsächlich durch die Konzentration und Viskosität der Imprägnierlösung steuerbar ist.

Besonders vorteilhaft können erfindungsgemäß die nach der vorstehenden Verfahrensweise erhältlichen geträgerten edelmetallhaltigen Katalysatoren für die Herstellung von 3-Methylbut-2-en-1-al aus 3-Methylbut-3-en-1-ol eingesetzt werden. Das Produkt ist auch unter dem Trivialnamen Prenal (MBA), das Edukt unter dem Trivialnamen Isoprenol (MBE), bekannt.

Bei dieser besonders bevorzugten Verwendung wird die Umsetzung vorzugsweise in einem Rohrbündelreaktor, wie er z. B. in der EP 0 881 206 A1 beschrieben ist, durchgeführt. Wegen weiterer Einzelheiten zur Reaktorgeometrie sei hier explizit auf EP 0 881 206 A1, Seite 2, Zeilen 37 bis 45 und Seite 5, Zeilen 40 bis 43 und die EP 0 244 632 A2, Figuren 1 bis 3 verwiesen.

Durch die erfindungsgemäße Verwendung der wie vorstehend beschrieben erhältlichen silberhaltigen mit Steatit geträgerten Katalysatoren ist es möglich, Prenal aus Isoprenol unter milden Temperaturbedingungen mit guter Ausbeute und guter Selektivität zu erhalten. Bei der Umsetzung von Isoprenol mit dem wie vorstehend beschrieben erhältlichen edelmetallhaltigen geträgerten Katalysator entsteht ein Reaktionsgemisch aus 3-Methylbut-3-en-1-al (IMBA) und 3-Methylbut-2-en-1-al (MBA).

Bei der Aufarbeitung des Reaktionsgemisches wird in einer ersten Stufe das gewünschte Reaktionsprodukt von nicht umgesetztem Edukt destillativ getrennt. Um diese Destillation wirtschaftlich vorteilhaft durchführen zu können, macht man sich vorteilhaft ein Azeotrop zu Nutze, welches aus 70% 3-Methylbut-3-en-1-al und 30% 3-Methylbut-2-en-1-al besteht.

Nach der erfindungsgemäßen Verwendung des wie vorstehend beschrieben erhältlichen mit Steatit geträgerten silberhaltigen Katalysators kann Prenal in guter Ausbeute bei niedrigeren Temperaturen und mit guter Selektivität aus Isoprenol hergestellt werden.

Es lassen sich im Rahmen der vorliegenden Erfindung z.B. für die Reaktion MBE zu MBA bei Temperaturen von 350 bis 385°C Ausbeuten von größer als 45%, insbesondere von 48% und mehr, sowie Selektivitäten von größer als 75%, insbesondere von 80% und mehr erreichen.

In einer weiteren vorteilhaften Variante können erfindungsgemäß die nach der vorstehenden Verfahrensweise erhältlichen mit Steatit geträgerten silberhaltigen Katalysatoren für die Herstellung von Ethylenoxid aus Ethylen eingesetzt werden.

Es lassen sich im Rahmen der vorliegenden Erfindung z.B. für die Epoxidation von Ethylen zu Ethylenoxid bei Temperaturen von 245 bis 270°C Ausbeuten von von 5 bis 10%, insbesondere von größer als 6% (z.B. 6,7%), beispielsweise von 6 bis 8% im einfachen Durchgang erreichen, sowie Selektivitäten von größer als 70%, insbesondere von 73 bis 82% und mehr erreichen.

Selbstverständlich können die erfindungsgemäßen Katalysatoren auch für andere Reaktionen als nur die genannten verwendet werden, insbesondere für Oxidationen im allgemeinen.

Demgemäß umfasst die vorliegende Erfindung auch allgemein die Verwendung der erfindungsgemäßen Katalysatoren für Oxidationsreaktionen.

Ein Vorteil der vorliegenden Erfindung ist demnach, dass die Herstellung der Katalysatoren sehr einfach und gut zu steuern (variieren) ist. Vorteilhaft ist weiterhin, dass mit den erfindungsgemäßen Katalysatoren sehr gute Ausbeuten und Selektivitäten erzielt werden.

Weiterer Vorteil der vorliegenden Erfindung ist es, dass Katalysatoren erhalten werden, die selbst bei einer deutlichen Erhöhung der Gasmenge um 10 oder sogar 20% keine Leistungseinbrüche zeigen, sondern in ihrer Selektivität und Konversion konstant bleiben.

In einer weniger bevorzugten, aber möglichen Variante der vorliegenden Erfindung können Epoxidation und oxidative Dehydrierung gleichzeitig erfolgen.

Es ist selbstverständlich möglich, die erfindungsgemäßen Katalysatoren sowohl in Verfahren einzusetzen, die im einfachen Durchgang gefahren werden, als auch in Verfahren, die in Kreisfahrweise gefahren werden.

Die verschiedenen Ausgestaltungen der vorliegenden Erfindung, z.B. diejenigen der verschiedenen abhängigen Ansprüche, können dabei in beliebiger Art und Weise miteinander kombiniert werden.

Die Erfindung wird nun unter Bezugnahme auf die folgenden nicht-limitierenden Beispiele erläutert.

Beispiele:

Beispiel 1: Herstellung einer Lösung von kolloidalem Silber

30,02 g kolloidales Silber (Fluka, Bestell-Nr.; 85131) wurden portionsweise unter Rühren zu 120,01 g destilliertem Wasser gegeben und 1h nachgerührt. Es bildete sich eine grünbraune, kolloidale Lösung mit einem Massenanteil von ca. 13,8 Gew.-% Silber.

Beispiel 2: Herstellung eines erfindungsgemäß geträgerten Katalysators

100 g Steatit-Kugeln eines mittleren Durchmessers von 1,8-2,2 mm (Fa. Ceramtec) wurden in einem 250 ml Becherglas vorgelegt. Anschließend wurden 37,0 g der gemäß Beispiel 1 hergestellten kolloidalen Silberlösung zugegeben und mit einem Spatel vermischt. Nach 45 Minuten Standzeit mit gelegentlichem Rühren mit dem Spatel wurden die beschichteten Kugeln in ein quadratisches Netz mit der Kantenlänge 21,5 cm, Maschenweite 1,5 mm überführt und in einem Ofen (Horo, Typ 112) für 12 Minuten bei 250°C unter einem Luftstrom von 8 $m^3$/h und einem apparativ bedingten Überdruck von 1 $mbar_{ü}$ über Atmosphärendruck getrocknet. Die beschichteten Steatitkugeln wurden anschließend bei 450°C für eine Stunde im Muffelofen (Nabertherm) unter Luft kalziniert und danach wieder auf Raumtemperatur abgekühlt.

Die Auftragung der Aktivmasse auf den Katalysator betrug 1,96%.

Beispiel 3: Katalysatortestung - Herstellung von Prenal

Eine Schüttung von 10 ml des nach Beispiel 2 hergestellten Katalysators wurde in einen Quarzglasreaktor gefüllt. Dann wurde die Reaktion (Herstellung von 3-Methylbut-2-en-1-al aus 3-Methylbut-3-en-1-ol) durchgeführt, indem 110 g/h MBE und 50 l/h Luft über einen Dünnschichtverdampfer verdampft wurden, wobei die Temperatur so eingestellt wurde, dass der MBE-Umsatz bei 60% lag.

Der kolloidale Silberkatalysator zeigte auch nach über 1800 Stunden Betriebszeit und über vielfache Abbrennzyklen, sowie bei einer Erhöhung der GHSV (Gas Hourly Space Velocity) um 10% und um 20% stabile Leistungsdaten von 60% MBE-Umsatz bei 80% MBA + IMBA Selektivität und Ausbeuten von 48%.

Damit ist der nach Beispiel 2 hergestellte Katalysator dem konventionellen Katalysator, mit dem sich lediglich Ausbeuten von 45% erzielen lassen, deutlich überlegen.

Beispiel 4:Katalysatortestung - Herstellung von Ethylenoxid

Zunächst wurden analog zu den obigen Beispielen 1 und 2 ein geträgerter Katalysator hergestellt, indem kolloidale Silberlösung enthaltend Salze verschiedener Promotoren auf alpha-Aluminiumoxid als Trägermaterial abgeschieden wurde, wobei der Katalysator für 12 Minuten bei 280°C thermisch behandelt und nicht nachkalziniert wurde. Es wurde ein geträgerter Katalysator mit einem Gehalt von 15,5 Gew.-% Silber erhalten, der durch verschiedene Promotoren (Li - 175 ppm, Cs - 430 ppm, W - 140 ppm, Re - 270 ppm, S - 38 ppm;) modifiziert ist.

Als Rohrreaktor wurde ein Doppelmantelreaktor aus Edelstahl mit einem Innendurchmesser von 6 mm verwendet. Die Länge des Rohrreaktors betrug 2200 mm. Als Katalysatorschüttung wurden 29,2 g Splitt mit einer Korngröße von 0,6 bis 0,9 mm des geträgerten Katalysators verwendet. Die Schüttungslänge betrug ca. 110 cm.

Dem Reaktionsraum wurde von oben nach unten strömend ein Reaktionsgaseingangsgemisch zugeführt, das nachfolgende Zusammensetzung aufwies:

| | |
|---|---|
| 35 Vol.-% | Ethylen, |
| 7 Vol.-% | molekularer Sauerstoff, |
| 1 Vol.-% | Kohlenstoffdioxid, |
| 0,15 Vol.-% | Wasser, |
| 2,6 - 2,9 Vol.-ppm | Ethylchlorid (Moderator) und als Restmenge bis zu 100 Vol -% Methan. |

Der Eingangsdruck des Reaktionsgasgemischs beim Eintritt in den Reaktionsraum betrug 16 bar absolut. Die Belastung der ca. 110 cm langen Katalysatorsplittschüttung mit dem Reaktionsgaseingangsgemisch betrug 4750 $h^{-1}$. Als Temperiermedium wurde im zwischen den beiden Mänteln befindlichen Zwischenraum ein Wärmeträgeröl vom Typ AP 100 Silikonöl der Firma Wacker Chemie, München, geführt. Das Wärmeträgeröl wurde am unteren Ende des Rohrreaktors mit einer Eingangstemperatur $T_W^{Ein}$ zugeführt und strömte von unten nach oben, wo es aus dem Mantelzwischenraum herausströmte. $T_W^{Ein}$ wurde so gewählt und kontinuierlich angepasst, dass der Gehalt des Reaktionsgasgemischs beim Verlassen des Reaktionsraums stetig 2,7 Vol.-% Ethylenoxid betrug. Der Gehalt des Reaktionsgaseingangsgemischs an Ethylenchlorid wurde über die Laufzeit so erhöht, dass zu jedem Betriebszeitpunk die maximale Selektivität der Ethylenoxidzielproduktbildung gewährleistet war.

Nach 150 Betriebsstunden wurde folgendes Versuchsergebnis erhalten:

$$T_W^{Ein} = 252°C; S^{Ethylenoxid} = 74\% \ (S^{Ethylenoxid} = \text{Selektivität zu Ethylenoxid}).$$

**EP 2 448 669 B1**

**Patentansprüche**

1. Verwendung eines geträgerten edelmetallhaltigen Katalysators, erhältlich durch

    a) Aufbringen von kolloidalem Silber in Form einer kolloidalen Lösung, gegebenenfalls in Mischung mit als Promotoren wirkenden Zusätzen, auf Steatit als Trägermaterial,
    b1) Trocknen des erhaltenen Produkts bei 150 bis 350°C, oder
    b2) Trocknen des erhaltenen Produkts bei 150 bis 350°C und anschließende Kalzinierung bei 350 bis 550°C,

    zur Epoxidation oder oxidativen Dehydrierung.

2. Verwendung eines geträgerten edelmetallhaltigen Katalysators, erhältlich durch

    a) Aufbringen von kolloidalem Silber in Form einer kolloidalen Lösung, gegebenenfalls in Mischung mit als Promotoren wirkenden Zusätzen, auf Steatit als Trägermaterial und
    anschließende Kalzinierung bei 350 bis 550°C,

    wobei das in a) erhaltene Produkt direkt auf Kalzinierungstemperatur erhitzt wird,
    zur Epoxidation oder oxidativen Dehydrierung.

3. Verwendung nach Anspruch 1 oder 2, zur Herstellung von olefinisch ungesättigten Carbonylverbindungen aus olefinisch ungesättigten Alkoholen durch oxidative Dehydrierung.

4. Verwendung nach Anspruch 1 bis 3, **dadurch gekennzeichnet, dass** 3-Methylbut-2-en-1-al und 3-Methylbut-3-en-1-al aus 3-Methylbut-3-en-1-ol hergestellt wird.

5. Verfahren zur Herstellung eines geträgerten edelmetallhaltigen Katalysators zur Epoxidation oder oxidativen Dehydrierung, **dadurch gekennzeichnet, dass**

    a) kolloidales Silber in Form einer kolloidalen Lösung, ggf. in Mischung mit als Promotoren wirkenden Zusätzen, auf Steatit als Trägermaterial aufgebracht wird,
    b1) das erhaltene Produkt bei 150 bis 350°C getrocknet wird, oder
    b2) das erhaltene Produkt bei 150 bis 350°C getrocknet und anschließend bei 350 bis 550°C kalziniert wird.

6. Verfahren zur Herstellung eines geträgerten edelmetallhaltigen Katalysators zur Epoxidation oder oxidativen Dehydrierung, **dadurch gekennzeichnet, dass**

    a) kolloidales Silber in Form einer kolloidalen Lösung, ggf. in Mischung mit als Promotoren wirkenden Zusätzen, auf Steatit als Trägermaterial aufgebracht wird, und

    das in a) erhaltene Produkt anschließend bei bei 350 bis 550°C kalziniert wird,
    wobei das in a) erhaltene Produkt direkt auf Kalzinierungstemperatur erhitzt wird.

7. Verfahren nach Anspruch 5 oder 6, **dadurch gekennzeichnet, dass** Schritt a) die Teilschritte

    a1) Vermischen von Steatit als Trägermaterial mit kolloidal gelöstem Silber,
    a2) optional Stehenlassen der resultierenden Mischung für einen Zeitraum von 1 bis 90 Minuten, gegebenenfalls unter kontinuierlichem oder zeitweisem Rühren
    a3) Abtrennen des erhaltenen Produkts, bevorzugt durch Filtrieren und/oder Abdampfen,

    umfaßt.

8. Geträgerter edelmetallhaltiger Katalysator, erhältlich durch

    a) Aufbringen von kolloidalem Silber in Form einer kolloidalen Lösung, gegebenenfalls in Mischung mit als Promotoren wirkenden Zusätzen, auf Steatit als Trägermaterial,
    b1) Trocknen des erhaltenen Produkts bei 150 bis 350°C, oder
    b2) Trocknen des erhaltenen Produkts bei 150 bis 350°C und anschließende Kalzinierung bei 350 bis 550 °C.

9. Geträgerter edelmetallhaltiger Katalysator, erhältlich durch

a) Aufbringen von kolloidalem Silber in Form einer kolloidalen Lösung, gegebenenfalls in Mischung mit als Promotoren wirkenden Zusätzen, auf Steatit als Trägermaterial und

anschließende Kalzinierung bei 350 bis 550°C,
wobei das in a) erhaltene Produkt direkt auf Kalzinierungstemperatur erhitzt wird.

**Claims**

1. The use of a supported noble metal-comprising catalyst which can be obtained by

a) application of colloidal silver in the form of a colloidal solution, optionally in admixture with additives acting as promoters, to steatite as support material,
b1) drying of the resulting product at from 150 to 350°C, or
b2) drying of the resulting product at from 150 to 350°C and subsequent calcination at from 350 to 550°C

for epoxidation or oxidative dehydrogenation.

2. The use of a supported noble metal-comprising catalyst which can be obtained by

a) application of colloidal silver in the form of a colloidal solution, optionally in admixture with additives acting as promoters, to steatite as support material and subsequent calcination at from 350 to 550°C, where the product obtained in a) is heated directly to calcination temperature,

for epoxidation or oxidative dehydrogenation.

3. The use according to claim 1 or 2 for preparing olefinically unsaturated carbonyl compounds from olefinically unsaturated alcohols by oxidative dehydrogenation.

4. The use according to any of claims 1 to 3, wherein 3-methylbut-2-en-1-al and 3-methylbut-3-en-1-al are prepared from 3-methylbut-3-en-1-ol.

5. A process for producing a supported noble metal-comprising catalyst for epoxidation or oxidative dehydrogenation, wherein

a) colloidal silver in the form of a colloidal solution, optionally in admixture with additives acting as promoters, is applied to steatite as support material,
b1) the product obtained is dried at from 150 to 350°C, or
b2) the product obtained is dried at from 150 to 350°C and subsequently calcined at from 350 to 550°C.

6. A process for producing a supported noble metal-comprising catalyst for epoxidation or the oxidative dehydrogenation, wherein

a) colloidal silver in the form of a colloidal solution, optionally in admixture with additives acting as promoters, is applied to steatite as support material, and

the product obtained in a) is subsequently calcined at from 350 to 550°C, where the product obtained in a) is heated directly to calcination temperature.

7. The process according to claim 5 or 6, wherein step a) comprises the substeps

a1) mixing of steatite as support material with colloidally dissolved silver,
a2) optionally ageing of the resulting mixture by allowing it to stand for a period of from 1 to 90 minutes, optionally with continuous or intermediate stirring,
a3) isolation of the resulting product, preferably by filtration and/or evaporation.

**8.** A supported noble metal-comprising catalyst which can be obtained by

a) application of colloidal silver in the form of a colloidal solution, optionally in admixture with additives acting as promoters, to steatite as support material,
b1) drying of the resulting product at from 150 to 350°C, or
b2) drying of the resulting product at from 150 to 350°C and subsequent calcination at from 350 to 550°C.

**9.** A supported noble metal-comprising catalyst which can be obtained by

a) application of colloidal silver in the form of a colloidal solution, optionally in admixture with additives acting as promoters, to steatite as support material and

subsequent calcination at from 350 to 550°C,
where the product obtained in a) is heated directly to calcination temperature.


**Revendications**

**1.** Utilisation d'un catalyseur supporté contenant un métal noble, pouvant être obtenu par :

a) l'application d'argent colloïdal sous la forme d'une solution colloïdale, éventuellement en mélange avec des additifs agissant en tant que promoteurs, sur de la stéatite en tant que matériau support,
b1) le séchage du produit obtenu à une température de 150 à 350 °C, ou
b2) le séchage du produit obtenu à une température de 150 à 350 °C, puis la calcination à une température de 350 à 550 °C,

pour l'époxydation ou la déshydrogénation oxydative.

**2.** Utilisation d'un catalyseur supporté contenant un métal noble, pouvant être obtenu par :

a) l'application d'argent colloïdal sous la forme d'une solution colloïdale, éventuellement en mélange avec des additifs agissant en tant que promoteurs, sur de la stéatite en tant que matériau support, puis

la calcination à une température de 350 à 550 °C,
le produit obtenu en a) étant directement porté à la température de calcination,
pour l'époxydation ou la déshydrogénation oxydative.

**3.** Utilisation selon la revendication 1 ou 2, pour la fabrication de composés de carbonyle oléfiniquement insaturés à partir d'alcools oléfiniquement insaturés par déshydrogénation oxydative.

**4.** Utilisation selon les revendications 1 à 3, **caractérisée en ce que** du 3-méthylbut-2-én-1-al et du 3-méthylbut-3-én-1-al sont fabriqués à partir de 3-méthylbut-3-én-1-ol.

**5.** Procédé de fabrication d'un catalyseur supporté contenant un métal noble pour l'époxydation ou la déshydrogénation oxydative, **caractérisé en ce que**

a) de l'argent colloïdal est appliqué sous la forme d'une solution colloïdale, éventuellement en mélange avec des additifs agissant en tant que promoteurs, sur de la stéatite en tant que matériau support,
b1) le produit obtenu est séché à une température de 150 à 350 °C, ou
b2) le produit obtenu est séché à une température de 150 à 350 °C, puis calciné à une température de 350 à 550 °C.

**6.** Procédé de fabrication d'un catalyseur supporté contenant un métal noble pour l'époxydation ou la déshydrogénation oxydative, **caractérisé en ce que**

a) de l'argent colloïdal est appliqué sous la forme d'une solution colloïdale, éventuellement en mélange avec des additifs agissant en tant que promoteurs, sur de la stéatite en tant que matériau support, puis

le produit obtenu en a) est calciné à une température de 350 à 550 °C,
le produit obtenu en a) étant directement porté à la température de calcination.

7.  Procédé selon la revendication 5 ou 6, **caractérisé en ce que** l'étape a) comprend les étapes partielles suivantes :

    a1) le mélange de stéatite en tant que matériau support avec de l'argent dissous sous forme colloïdale,
    a2) éventuellement le repos du mélange résultant pendant une durée de 1 à 90 minutes, éventuellement sous agitation continue ou intermittente,
    a3) la séparation du produit obtenu, de préférence par filtration et/ou évaporation.

8.  Catalyseur supporté contenant un métal noble, pouvant être obtenu par :

    a) l'application d'argent colloïdal sous la forme d'une solution colloïdale, éventuellement en mélange avec des additifs agissant en tant que promoteurs, sur de la stéatite en tant que matériau support,
    b1) le séchage du produit obtenu à une température de 150 à 350 °C, ou
    b2) le séchage du produit obtenu à une température de 150 à 350 °C, puis la calcination à une température de 350 à 550 °C.

9.  Catalyseur supporté contenant un métal noble, pouvant être obtenu par :

    a) l'application d'argent colloïdal sous la forme d'une solution colloïdale, éventuellement en mélange avec des additifs agissant en tant que promoteurs, sur de la stéatite en tant que matériau support, puis

    la calcination à une température de 350 à 550 °C,
    le produit obtenu en a) étant directement porté à la température de calcination.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 2020865 B
- EP 881206 A
- DE 2715209 A
- EP 357292 A
- EP 619142 A
- DE 102008014910
- GB 940710 A
- DE 4010182 A1
- EP 1209121 A1
- EP 0112261 A1
- US 4816606 A
- EP 0881206 A1
- EP 0244632 A2

**In der Beschreibung aufgeführte Nicht-Patentliteratur**

- **ABAD et al.** Catalyst Parameters Determining Activity and Selectivity of Supported Gold Nanoparticles for the Aerobic Oxidation of Alcohols: The Molecular Reaction Mechnism. *Chem. Eur. J,* 2008, vol. 14, 212-222
- **W. SCHOLTAN ; H. LANGE.** *Kolloid-Z. und Z.-Polymere,* 1972, vol. 250, 782-796